(19) Europäisches Patentamt
European Patent Office
Office européen des brevets

(11) **EP 4 585 675 A1**

(12) **EUROPEAN PATENT APPLICATION**
published in accordance with Art. 153(4) EPC

(43) Date of publication:
**16.07.2025 Bulletin 2025/29**

(21) Application number: 23868391.6

(22) Date of filing: 07.08.2023

(51) International Patent Classification (IPC):
**C12M 1/36** (2006.01)   **C12M 1/06** (2006.01)
**C12M 1/00** (2006.01)

(52) Cooperative Patent Classification (CPC):
**C12M 1/00; C12M 1/06; C12M 1/36**

(86) International application number:
**PCT/KR2023/011621**

(87) International publication number:
**WO 2024/063332 (28.03.2024 Gazette 2024/13)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.09.2022 KR 20220119331**

(71) Applicant: **CJ CheilJedang Corporation**
**Seoul 04560 (KR)**

(72) Inventors:
• **CHO, Jae-Hoon**
  **Seoul 04560 (KR)**
• **PARK, Chanhun**
  **Seoul 04560 (KR)**
• **KIM, Jun-Woo**
  **Seoul 04560 (KR)**

(74) Representative: **Meissner Bolte Partnerschaft mbB**
**Patentanwälte Rechtsanwälte**
**Postfach 86 06 24**
**81633 München (DE)**

(54) **CFD-BASED OPTIMIZATION METHOD AND SYSTEM OF FERMENTATION TANK FOR AMINO ACID FERMENTATION**

(57)    A CFD-based optimization method for a fermenter for amino acid fermentation according to an embodiment of the present invention includes: a first step of setting an objective variable, a constraint variable, and a design variable with respect to an optimization objective of at least one impeller fermenter; a second step of generating a three-dimensional shape for a flow region of the impeller fermenter for each condition of the constraint variable and the design variable according to the objective variable; a third step of calculating a primary value, which is at least one of oxygen diffusion coefficient, liquid density, liquid viscosity, gas hold-up, bubble diameter, energy dissipation rate, and torque, by computational fluid dynamics analysis for the flow region based on information about the three-dimensional shape; a fourth step of calculating secondary values, which are an oxygen transfer coefficient and a power consumption ratio (power ratio), using the primary value; and a fifth step of repeating the second, third, and fourth steps while varying the design variable of the first step in various ways, and finding an optimal point based on a comparison value obtained by comparing the secondary values before and after the variation.

FIG. 1

Set objective variable, constraint variable, and design variable — ST1

Generate three-dimensional shape for flow region of impeller fermenter for each condition — ST2

Calculate primary value by computational fluid dynamics analysis for flow region based on three-dimensional shape information — ST3

Calculate secondary values, which oxygen transfer coefficient and power ratio, using primary value — ST4

Calculate tertiary value using secondary values — ST41

Find optimal point by comparison value obtained by comparing secondary values before and after variation when varying design variable — ST5

Find optimal point by comparison value obtained by comparing tertiary values before and after variation — ST51

## Description

### [Technical Field]

The present invention relates to a CFD-based optimization method and system for a fermenter for amino acid fermentation.

### [Background Art]

In the field of biology, methods for improving the efficiency of fermenters include biological microbial modification and microbial recirculation. Microbial modification and microbial recirculation methods increase microbial productivity and microbial concentration. During this process, the oxygen demand directly related to microbial amino acid production increases.

In this case, various variable, such as high concentration oxygen, aeration rate, rotational speed (RPM), and impeller type, are considered to ensure the rapid transfer of oxygen from the supplied air to the culture medium.

There are also methods, such as increasing the diameter or rotational speed (RPM) of the impeller, to ensure the rapid transfer of oxygen, but these methods consume a significant amount of energy. Therefore, it is necessary to find operating conditions suitable for the current situation by considering both oxygen transfer and energy consumption.

As such, many experiments are needed to analyze oxygen transfer and energy consumption for each of various operating conditions. In the laboratory, experiments can be conducted by fabricating small-scale fermenters with varying heights, diameters, and impeller sizes and shapes, as well as based on different operating conditions.

However, since fermenters used in production sites are large-scale, changing the structure of these fermenters or conducting experiments under different operating conditions requires halting production and incurs significant costs. Therefore, there are limitations to conducting experiments with large-scale fermenters.

### [Disclosure]

### [Technical Problem]

An object of the present invention is to provide a CFD-based optimization method for a fermenter for amino acid fermentation, which simulates a flow phenomenon within the fermenter based on computational fluid dynamics.

Another object of the present invention is to provide a CFD-based optimization method for a fermenter for amino acid fermentation, which overcomes physical limitations and predicts an oxygen transfer coefficient and power consumption for amino acid fermentation within the fermenter for amino acid fermentation.

Still another object of the present invention is to provide a CFD-based optimization method for a fermenter for amino acid fermentation for finding an optimal condition for efficiently supplying oxygen to a fermentation medium within the fermenter for amino acid fermentation.

Yet another object of the present invention is to provide a CFD-based optimization system for a fermenter for amino acid fermentation, which applies the above CFD-based optimization method for a fermenter for amino acid fermentation.

### [Technical Solution]

A CFD-based optimization method for a fermenter for amino acid fermentation according to an embodiment of the present invention includes: setting an objective variable, a constraint variable, and a design variable with respect to an optimization objective of the fermenter having at least one impeller; generating a three-dimensional shape for a flow region of the impeller fermenter for each condition of the constraint variable and the design variable according to the objective variable; calculating a primary value, which is at least one of oxygen diffusion coefficient, liquid density, liquid viscosity, gas hold-up, bubble diameter, energy dissipation rate, and torque, by computational fluid dynamics analysis for the flow region based on information about the three-dimensional shape; calculating secondary values, which are an oxygen transfer coefficient and a power consumption ratio (power ratio), using the primary value; and repeating the generating the three-dimensional shape, the calculating the primary value , and the calculating the secondary values while varying the design variable of the setting in various ways, and finding an optimal point based on a comparison value obtained by comparing the secondary values before and after the variation.

In the setting, at least one of the oxygen transfer coefficient and the power ratio may be selected as the objective variable, considering a current situation.

In the setting, the constraint variable may include at least one of a height of the fermenter, a diameter of the fermenter, a shape of a sparger, an aeration rate, and an operating volume of the fermenter.

In the setting, the design variable may be set as a computational fluid dynamics simulation condition within a range where variations to the fermenter are possible.

In the setting, the design variable may include at least one of a rotational speed (rpm) of the impeller, a shape of the impeller, the number of impellers, a diameter of the impeller, and an installation height of the impeller.

In the setting, a maximum height of the impeller may be set within an operating volume height of the fermenter.

The calculating of the secondary values may include calculating a tertiary value, which is a ratio of the oxygen transfer coefficient per power ratio (kLa/power ratio), using the oxygen transfer coefficient and the power ratio.

The finding of the optimal point may includefinding the optimum point based on a comparison value obtained by comparing the tertiary values before and after the variation.

A CFD-based optimization system for a fermenter for amino acid fermentation according to an embodiment of the present invention includes a simulation unit configured to perform simulation by the CFD-based optimization method for a fermenter for amino acid fermentation; a fermenter optimized by the simulation unit and applied to a field; a monitoring sensor configured to monitor error data deviating from an optimal state of the fermenter during an operation; and a control unit configured to compare whether the error data monitored by the monitoring sensor exceeds an allowable error range and to notify an administrator of a result of the comparison.

The fermenter may include a first-stage impeller, a second-stage impeller, and a third-stage impeller installed on a shaft driven by a motor, spaced apart at predetermined intervals upward from a lower end, a first baffle and a second baffle provided on both sides of a diametrically inner wall, and a sparger provided below the first-stage impeller to blow air.

In this way, an embodiment of the present invention can adjust the optimal impeller height according to the amino acid type and operating conditions of a fermenter to which at least one impeller is applied through computational fluid dynamics analysis for the flow region of the fermenter based on the three-dimensional shape information.

An embodiment of the present invention can produce various amino acids according to demand for each product, and can improve productivity and reduce power consumption by adjusting the impeller height of the fermenter, in a production plant where production volume needs to be adjusted.

An embodiment of the present invention enables the calculation of fluid phenomena in a fermenter through computational fluid dynamics analysis and the objective verification of the oxygen transfer coefficient and power consumption, thereby making it possible to calculate the effect of increasing the oxygen transfer coefficient in relation to power consumption.

Therefore, economic benefits can be expected. That is, an embodiment of the present invention can reflect the structural characteristics of a fermenter used in an industrial field through computational fluid dynamics analysis, and implement optimization through prediction of the oxygen transfer coefficient and power consumption of the fermenter.

**[Description of the Drawings]**

FIG. 1 is a flowchart of a CFD-based optimization method for a fermenter for amino acid fermentation according to an embodiment of the present invention.

FIG. 2 is a basic configuration view of a fermenter for amino acid fermentation according to an embodiment of the present invention.

FIG. 3 is a photograph of impellers used in a first experimental example of a CFD-based optimization method for a fermenter for amino acid fermentation according to an embodiment of the present invention.

FIGS. 4 to 6 are perspective views showing relationships of impellers within a fermenter generated in a simulation of a CFD-based optimization method for a fermenter for amino acid fermentation according to an embodiment of the present invention.

FIG. 7 is a block diagram of a CFD-based optimization system for a fermenter for amino acid fermentation according to an embodiment of the present invention.

**[Mode for Invention]**

The present invention will be described more fully hereinafter with reference to the accompanying drawings, in which embodiments of the invention are shown. As those skilled in the art would realize, the described embodiments may be modified in various different ways, all without departing from the spirit or scope of the present invention. The drawings and description are to be regarded as illustrative in nature and not restrictive. Like reference numerals designate like elements throughout the specification.

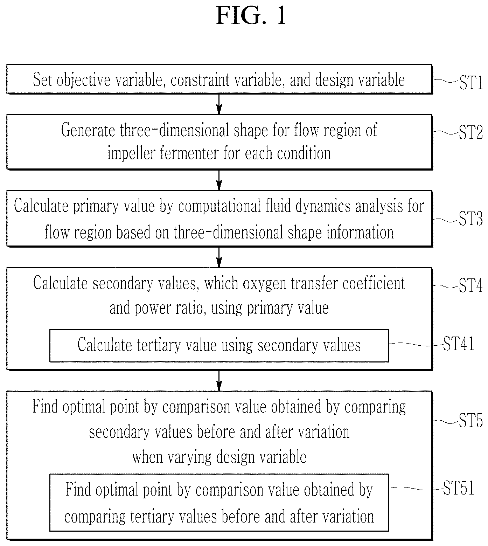

FIG. 1 is a flowchart of a CFD-based optimization method for a fermenter for amino acid fermentation according to an embodiment of the present invention. Referring to FIG. 1, a CFD-based optimization method of an embodiment includes a first step (ST1), a second step (ST2), a third step (ST3), a fourth step (ST4), and a fifth step (ST5).

The first step (ST1) involves setting an objective variable, a constraint variable, and a design variable with respect to an optimization objective of a fermenter having at least one impeller. The first step (ST1) may involve selecting at least one of an oxygen transfer coefficient (kLa) and a power consumption ratio (power ratio) as the objective variable, considering a current situation.

The first step (ST1) may involve at least one of a height of the fermenter, a diameter of the fermenter, a shape of a sparger, an aeration rate, and an operating volume of the fermenter, as the constraint variable.

The first step (ST1) may involve setting the design variable as a computational fluid dynamics simulation condition within a range where variations to the fermenter are possible. The first step (ST1) may involve at least one of a rotational speed (rpm) of the impeller, a shape of the impeller, the number of impellers, a diameter of the impeller, and an installation height of the impeller, as the design variable. The first step (ST1) may involve setting a maximum height of the impeller within an operating volume height of the fermenter.

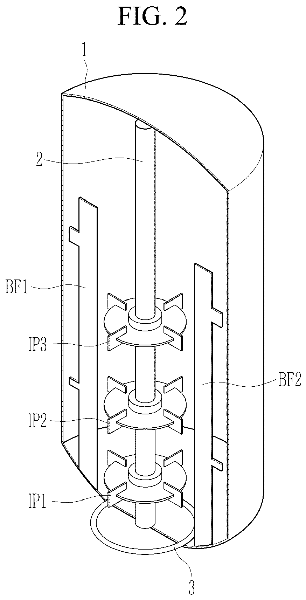

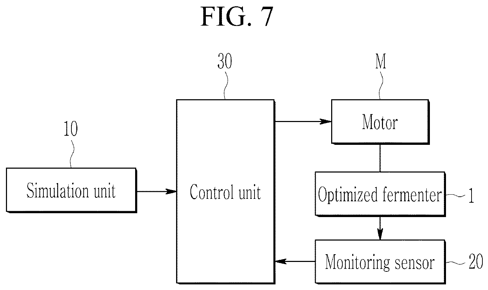

FIG. 2 is a basic configuration view of a fermenter for amino acid fermentation according to an embodiment of the present invention. Referring to FIG. 2, a fermenter 1 for amino acid fermentation includes a first-stage impeller IP1, a second-stage impeller IP2, and a third-stage impeller IP3 installed on a single shaft 2, spaced apart at predetermined intervals from a lower end toward an upper end, a first baffle BF1 and a second baffle BF2 provided on both sides of a diametrically inner wall, and a sparger 3 provided below the first-stage impeller IP1 to blow air. The shaft 2 rotationally operates by torque of a motor (M, see FIG. 7).

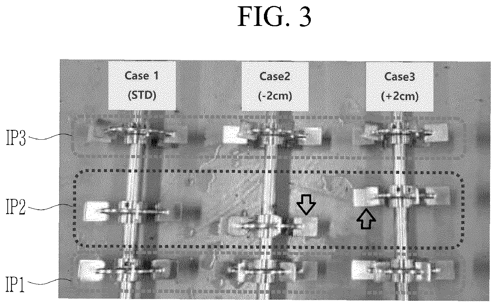

FIG. 3 is a photograph of impellers used in a first experimental example of a CFD-based optimization method for a fermenter for amino acid fermentation according to an embodiment of the present invention. Referring to FIG. 3, the first-stage impeller IP1, the second-stage impeller IP2, and the third-stage impeller IP3 are provided on a single shaft 2.

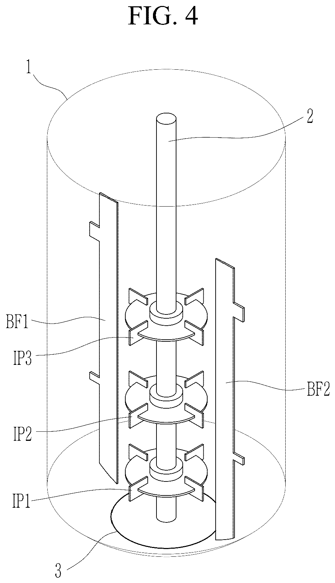

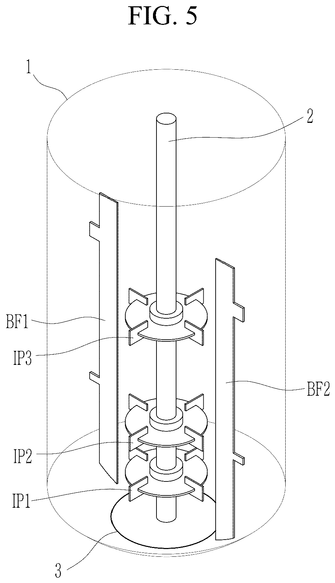

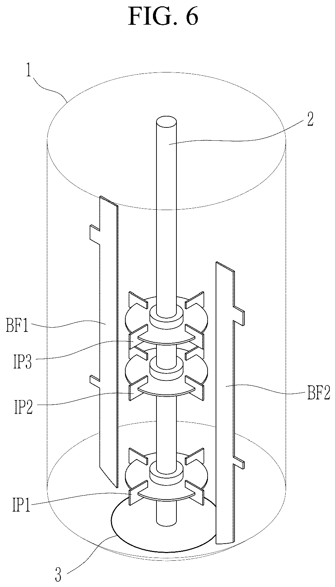

FIGS. 4 to 6 are perspective views showing relationships of impellers within a fermenter generated in a simulation of a CFD-based optimization method for a fermenter for amino acid fermentation according to an embodiment of the present invention.

Referring again to FIG. 1, the second step (ST2) involves generating a three-dimensional shape for a flow region of the impeller fermenter for each condition of the constraint variable and the design variable and according to the objective variable. FIGS. 4 to 6 show three-dimensional shapes generated for the flow region of the impeller fermenter.

The third step (ST3) involves calculating a primary value, which is at least one of oxygen diffusion coefficient, liquid density, liquid viscosity, gas hold-up, bubble diameter, energy dissipation rate, and torque, by computational fluid dynamics analysis for the flow region based on information about the three-dimensional shape.

A liquid is filled into the fermenter, and gas hold-up and bubbles are set by air blown in through a sparger 3. The torque refers to the rotational force of the motor (M) applied to the shaft 2 by the first-, second-, and third-stage impellers IP1, IP2, and IP3 due to the rotation of the shaft 2.

The fourth step (ST4) involves calculating secondary values, which are an oxygen transfer coefficient (kLa) and a power consumption ratio (power ratio), using the primary value. The oxygen transfer coefficient (kLa) is calculated using mathematical formula 1.

## (Mathematical Formula 1)

$$k_L a$$

oxygen transfer coefficient

$$k_L = \frac{2}{\sqrt{\pi}} \sqrt{D_L} \left( \frac{\varepsilon \rho}{\mu} \right)^{\frac{1}{4}} \ (m/s)$$

$D_L$ = oxygen diffusion coefficient ($kg/m^3$),
$\varepsilon$ = turbulent dissipation rate *(CFD, $m^2/s^3$)*
$\rho$ = liquid density ($kg/m^3$), $\mu$ = liquid viscosity ($kg/ms$)

$$a = \frac{6 \alpha_g}{d_b} \ (1/m)$$

$d_b$ = average diameter *(m)*, $\alpha_g$ = gas hold-up (-)

The power consumption (Pg) based on torque is calculated using mathematical formula 2, that is, rotations per second (N) and torque.

## (Mathematical Formula 2)

$$P_g = 2\pi N\tau$$

$$N = rps\ (1/s)$$

,

$\tau$ = torque

The torque is calculated during CFD operations. The power ratio is calculated as the power consumption (Pg) derived based on the torque.

The fifth step (ST5) involves sequentially repeating the second step (ST2), the third step (ST3), and the fourth step (ST4) while varying the design variable of the first step (ST1) in various ways, and finding an optimal point based on a comparison value obtained by comparing the secondary values before and after the variation, generated at each repetition.

In addition, the fourth step (ST4) may involve further calculating a tertiary value, which is a ratio of the oxygen transfer coefficient per power ratio (kLa/power ratio), using the secondary value.

In this case, the fifth step (ST5) may involve sequentially repeating the second step (ST2), the third step (ST3), and the fourth step (ST4) while varying the design variable of the first step (ST1) in various ways, and finding an optimal point based on a comparison value obtained by comparing the tertiary values before and after the variation, generated at each repetition.

Below, actual experimental examples and simulations will be described as examples.

Experimental Example 1 and Simulation 1

Referring to FIGS. 3 to 6, Experimental Example 1 and Simulation 1 according to a CFD-based optimization method of an embodiment will be described. FIGS. 4 to 6 are illustrations obtained in the second step (ST2). The second step (ST2) involves generating a three-dimensional shape for a flow region of the impeller fermenter for each condition of the first step (ST1).

The third step (ST3) involves calculating a primary value, which is at least one of oxygen diffusion coefficient, liquid density, liquid viscosity, gas hold-up, bubble diameter, energy dissipation rate, and torque, by computational fluid dynamics analysis for the flow region based on information about the three-dimensional shape.

Table 1 shows the oxygen transfer coefficient (kLa), the power ratio, and the ratio of oxygen transfer coefficient per power ratio (kLa/power ratio) according to the impeller height in a small-scale fermenter. As an example, a small-scale fermenter has a volume of greater than 1 L and less than 100 L.

[Table 1]

| Categorization | Unit | Case 1 (3 stages) | Case 2 (descentof 2 cm) | Case 3 (ascent of 2 cm) |
|---|---|---|---|---|
| Height | (mm) | 75 | 95 | 55 |
| Oxygen transfer coefficient (kLa) | (1/s) | 0.0452 | 0.0441 | 0.053 |
| Power ratio | | 1.00 | 0.98 | 1.10 |
| kLa/power ratio | | 0.0452 | 0.0450 | 0.0482 |
| Cultivation time ratio | - | 27 | 28 | 26 |
| Productivity ratio | - | 1.00 | 0.98 | 1.05 |

A simulation was performed using a small-scale fermenter. First, the size of the fermenter, the size of the impeller, and the rotational speed (RPM) of the impeller were fixed as the first variable, the height of the second-stage impeller IP2 as the second variable was varied (cases 1, 2, and 3), and the fermenter was simulated using CFD.

In Cases 1, 2, and 3, the first-stage impeller IP1, the second-stage impeller IP2, and the third-stage impeller IP3 were arranged from the lower side toward the upper side, and the position of the second stage impeller IP2 located in the middle was set to 75 mm as the reference, 55 mm lowered from the reference, and 95 mm raised from the reference.

Through this simulation, the oxygen transfer coefficient (kLa) and the power ratio, which are the third variables and the secondary values, were calculated in the fourth step (ST4).

As shown in Table 1 of the simulation results, the first-stage impeller IP1, the second-stage impeller IP2, and the third-stage impeller IP3 were arranged from the lower side toward the upper side, and it was confirmed in the fifth step (ST5) that the oxygen transfer coefficient (kLa) improved to 0.0441, 0.0452, and 0.0530 as the position of the second-stage impeller IP2 located in the middle was lowered to 95, 75, and 55 mm (case 3, case 1, and case 2), and the optimum point was found.

The reason for this result is that as the first-stage impeller IP1 and the second-stage impeller IP2 came closer to each other, the two first- and second-stage impellers IP1 and IP2 acted like one large impeller.

Based on the simulation results, the height of the second-stage impeller IP2 was changed as shown in Table 1, the actual fermenter was applied to the field, and cultivation was then conducted.

As a result, as shown in Table 2, it was confirmed that the fermentation time ratio decreased as the oxygen transfer coefficient (kLa), which is the secondary value, increased, and the productivity ratio improved according to the decreased fermentation time ratio. That is, it can be confirmed that that fermentation, which is closely related to oxygen, results in a decreased cultivation time ratio as the oxygen transfer coefficient (kLa) increases.

Referring again to FIG. 1, the fourth step (ST4) further includes a 41st (ST41) step of calculating a tertiary value, which is a ratio of the oxygen transfer coefficient (kLa) per the power ratio, by using the oxygen transfer coefficient (kLa) and the power ratio, which are the secondary values. In this case, the fifth step (ST5) further includes a 51st step (ST51) of finding an optimum point based on a comparison value obtained by comparing the tertiary values before and after variation.

The 51st step (ST51) involves finding the optimum point based on a comparison value obtained by comparing the tertiary values before and after variation when varying the design variable. The 51st step (ST51) involves repeating the second step (ST2), the third step (ST3), and the fourth and 41st steps (ST4 and ST41), and finding the optimum point based on a comparison value obtained by comparing the tertiary values before and after variation.

Referring to Table 1, as a result of the 41st step (ST41), when the position of the second-stage impeller IP2 in Case 1 is at the middle 75 mm, the ratio of oxygen transfer coefficient per power ratio (kLa/power ratio) is 0.0452. In Case 3, when the position of the second stage impeller IP2 is lowered to 55 mm, the ratio of the oxygen transfer coefficient per power ratio (kLa/power ratio) increases to 0.0482. In Case 2, when the position of the second stage impeller IP2 is raised to 95 mm, the ratio of oxygen transfer coefficient per power ratio (kLa/power ratio) decreases to 0.0450.

That is, as a result of the 51st step (ST51), as in Case 3, when the position of the second-stage impeller IP2 is lowered, it can be seen that the ratio of oxygen transfer coefficient per power ratio (kLa/power ratio), which is the tertiary value, increases, and this was confirmed in the 51st step (ST51), and the optimum point was found.

As a result, as shown in Table 1, the fermentation time ratio decreased and the productivity ratio improved as the ratio of oxygen transfer coefficient per power ratio (kLa/power ratio), which is the tertiary value, increased. That is, it can be confirmed that that fermentation, which is closely related to oxygen, results in a decreased cultivation time ratio as the ratio of oxygen transfer coefficient per power ratio (kLa/power ratio), which is the tertiary value, increases.

FIG. 7 is a block diagram of a CFD-based optimization system for a fermenter for amino acid fermentation according to an embodiment of the present invention. Referring to FIG. 7, a CFD-based optimization system of an embodiment includes a simulation unit 10, a fermenter 1, a monitoring sensor 20, and a control unit 30.

The simulation unit 10 is configured to implement a CFD-based optimization method for a fermenter for amino acid fermentation. The fermenter 1 is optimized using the CFD-based optimization method for a fermenter for amino acid fermentation of an embodiment and applied to the field. The monitoring sensor 20 may be configured as a camera, an infrared sensor, or the like capable of monitoring error data that deviates from the optimal state of the fermenter 1 during operation.

The control unit 30 compares whether the error data monitored by the monitoring sensor 20 exceeds an allowable error range and notifies an administrator of a result of the comparison. The control unit 30 controls the motor M of the fermenter 1 based on the data of the simulation unit 10 to drive the shaft 2 and the first-, second-, and third-stage impellers IP1, IP2, and IP3. At the same time, the control unit 30 determines whether the fermenter 1 is out of its optimized state through a detection signal from the monitoring sensor 20, thereby enabling the optimal fermentation state of the fermenter 1 to be maintained.

Below, various simulations and examples of field applications will be described.

Experimental Example 2 (Simulation 2 and Field Application)

Experimental Example 2 according to a CFD-based optimization method of an embodiment will be described. Table 2 shows the oxygen transfer coefficient (kLa) and productivity according to the height of the impeller within a large-scale fermenter. As an example, the large-scale fermenter has a volume of greater than 100,000 L and less than 700,000 L.

The experiment was conducted using the large-scale fermenter. First, the size of the fermenter, the size of the impeller, and the rotational speed (RPM) of the impeller were maintained as the first variable, the heights of the second- and third-stage impellers as the second variable were varied, and the fermenter was simulated using CFD.

Through this simulation, the power ratio and the oxygen transfer coefficient (kLa), which are the third variables and the secondary values, and the ratio of oxygen transfer coefficient per power ratio, which is the tertiary value, were calculated in the fourth and 41st steps (ST4 and ST41).

As shown in Table 2 of the simulation results, the highest oxygen transfer coefficient was confirmed in the fermenter 1 when the second-stage impeller and the third-stage impeller were installed at positions 10 cm and 50 cm higher than the standard (STD), respectively, as in fermenter 1. Seeing the heights of the second-stage and third-stage impellers in the fermenters 1 to 4, the oxygen transfer coefficient was high when the second-stage and third-stage impellers were at appropriate positions.

As a result of comparing productivity based on accumulated data from the fermenters 1 to 4, a proportional relationship between the oxygen transfer coefficient and the productivity was obtained. Then, the fermenter 4 was changed such that the second-stage impeller and the third-stage impeller were positioned at the same heights as in the fermenter 1, and the productivity was improved by 1 to 2%.

[Table 2]

| Categoriza tion | First stage | Second stage (cm) | Third stage (cm) | CFD kLa | Power ratio | kLa/ power ratio | Product ivity |
|---|---|---|---|---|---|---|---|
| Optimal conditions | STD | STD+10 | STD+50 | 0.1245 | 1.00 | 0.1245 | - |
| Fermenter 1 | STD | STD+10 | STD+50 | 0.1245 | 1.00 | 0.1245 | 1.029 |
| Fermenter 2 | STD | STD | STD | 0.1217 | 1.08 | 0.1123 | 1.000 |
| Fermenter 3 | STD | STD+30 | STD+60 | 0.1232 | 1.07 | 0.1151 | 1.014 |
| Fermenter 4 | STD | STD+60 | STD+110 | 0.1241 | 1.10 | 0.1128 | 1.017 |

Experimental Example 3 (Simulation 3)

Experimental Example 3 according to a CFD-based optimization method of an embodiment will be described. The target fermenter is a fermenter in which oxygen is insufficient, and the feed is thus reduced to prevent oxygen depletion and abnormal fermentation, which ultimately results in decreased fermentation productivity. The corresponding fermenter in Table 3 is a large-scale fermenter having a volume of greater than 100,000 L and less than 700,000 L.

A simulation was performed using the large-scale fermenter. In the simulation, the size of the fermenter, the size of the impeller, and the rotational speed (RPM) of the impeller were maintained as the first variable, the height of the second-stage impeller as the second variable was varied, and the fermenter was simulated using CFD.

Through this simulation, the power ratio and the oxygen transfer coefficient (kLa), which are the third variables and the secondary values, and the ratio of oxygen transfer coefficient per power ratio, which is the tertiary value, were calculated in the fourth and 41st steps (ST4 and ST41).

As shown in Table 3 of the simulation results, it was confirmed that the lower the height at which the second-stage impeller was installed, the higher the oxygen transfer coefficient in the fermenter.

[Table 3]

| Categorization | First stage | Second stage | Oxygen transfer coefficient | Power ratio | kLa/power ratio |
|---|---|---|---|---|---|
| Case 1 | STD | STD-40cm | 0.144 | 0.93 | 0.154 |
| Case 2 | STD | STD-30cm | 0.142 | 0.81 | 0.175 |
| Case 3 | STD | STD | 0.140 | 1.00 | 0.140 |
| Case 4 | STD | STD+110cm | 0.135 | 0.95 | 0.142 |
| Case 5 | STD | STD+160cm | 0.121 | 0.87 | 0.139 |

Experimental Example 4 (Simulation 4)

Experimental Example 4 according to a CFD-based optimization method of an embodiment will be described. The target fermenter is a fermenter in which the oxygen transfer coefficient needs to be increased due to oxygen depletion. The corresponding fermenter is a medium-scale fermenter having a volume of greater than 100 L and less than 100,000 L.

A simulation was performed using the medium-scale fermenter. For the corresponding fermenter in Table 4, the height of the impeller and the rotational speed of the impeller can be varied. In this simulation, the size of the fermenter and the size of the impeller were maintained as the first variable, the height and rotational speed (RPM) of the third-stage impeller as the second variable were varied, and the fermenter was simulated using CFD.

Through this simulation, the power ratio and the oxygen transfer coefficient (kLa), which are the third variables, and the ratio of oxygen transfer coefficient per power ratio, which is the tertiary value, were calculated in the fourth and 41st steps (ST4 and ST41).

As a result, simply changing the height of the impeller had a minimal effect, with the oxygen transfer coefficient varying by about 1%. Additionally, a simulation was conducted by varying the rotational speed (RPM) of the impeller, which can be varied, and it was confirmed that the oxygen transfer coefficient increased by 6%.

[Table 4]

| Categorization | First and second stages | Third stage | Ratio of rotational speeds | Oxygen transfer coefficient | Power ratio | kLa/power ratio |
|---|---|---|---|---|---|---|
| Case 1 | STD | STD-15cm | 1 | 0.1984 | 0.97 | 0.2047 |
| Case 2 | STD | STD | 1 | 0.2028 | 1.00 | 0.2028 |
| Case 3 | STD | STD+15cm | 1 | 0.2055 | 0.95 | 0.2153 |
| Case 4 | STD | STD+30cm | 1 | 0.2022 | 0.97 | 0.2084 |
| Case 5 | STD | STD | 1.1 | 0.2174 | 1.08 | 0.2007 |
| Case 6 | STD | STD+15cm | 1.1 | 0.2133 | 1.12 | 0.1910 |

Experimental Example 5 (Simulation 5)

Experimental Example 5 according to a CFD-based optimization method of an embodiment will be described. The corresponding fermenter in Table 5 is a large-scale fermenter having a volume of greater than 100,000 L and less than 700,000 L.

A simulation was performed using the large-scale fermenter. First, under the given conditions, the size of the fermenter, the size of the impeller, and the rotational speed of the impeller were maintained as the first variable, and the fermenter was simulated using CFD according to the amount of air supplied to the sparger as the second variable.

Through this simulation, the power ratio and the oxygen transfer coefficient (kLa), which are the third variables and the secondary values, and the ratio of oxygen transfer coefficient per power ratio were calculated in the fourth and 41st steps (ST4 and ST41).

As shown in Table 5 of the simulation results, as the aeration rate increases, the oxygen transfer coefficient increases, and the power ratio increases to a certain level and then decreases. Through this, it was confirmed that the efficiency was highest when the maximum amount of air was supplied within the operable region.

[Table 5]

| Categorization | Aeration | Oxygen transfer coefficient | Power ratio | kLa/power ratio |
|---|---|---|---|---|
| Case 1 | 1.00 | 0.1245 | 1.00 | 0.1245 |
| Case 2 | 1.05 | 0.1296 | 1.08 | 0.1196 |
| Case 3 | 1.10 | 0.1369 | 1.05 | 0.1305 |
| Case 4 | 1.15 | 0.1476 | 0.98 | 0.1513 |

While this invention has been described in connection with what is presently considered to be practical embodiments, it is to be understood that the invention is not limited to the disclosed embodiments. On the contrary, it is intended to cover various modifications and equivalent arrangements included within the spirit and scope of the appended claims.

(Description of Symbols)

| 1: | fermenter | 2: | shaft |
|---|---|---|---|
| 3: | sparger | 10: | simulation unit |
| 20: | monitoring sensor | 30: | control unit |

(continued)

| BF1: | first baffle | BF2: | second baffle |
| IP1: | first-stage impeller | IP2: | second-stage impeller |
| IP2: | third-stage impeller | M: | motor |

**Claims**

1. A CFD-based optimization method for a fermenter for amino acid fermentation comprising:

setting an objective variable, a constraint variable, and a design variable with respect to an optimization objective of the fermenter having at least one impeller;
generating a three-dimensional shape for a flow region of the fermenter for each condition of the constraint variable and the design variable according to the objective variable;
calculating a primary value, which is at least one of oxygen diffusion coefficient, liquid density, liquid viscosity, gas hold-up, bubble diameter, energy dissipation rate, and torque, by computational fluid dynamics analysis for the flow region based on information about the three-dimensional shape;
calculating secondary values, which are an oxygen transfer coefficient and a power ratio, using the primary value; and
repeating the generating the three-dimensional shape, the calculating the primary value, and the calculating the secondary values while varying the design variable of the setting in various ways, and finding an optimal point based on a comparison value obtained by comparing the secondary values before and after the variation.

2. The CFD-based optimization method of claim 1, wherein:
in the setting, at least one of the oxygen transfer coefficient and the power ratio is selected as the objective variable, considering a current situation.

3. The CFD-based optimization method of claim 1, wherein:
in the setting, the constraint variable comprises at least one of a height of the fermenter, a diameter of the fermenter, a shape of a sparger, an aeration rate, and an operating volume of the fermenter.

4. The CFD-based optimization method of claim 1, wherein:
in the setting, the design variable is set as a computational fluid dynamics simulation condition within a range where variations to the fermenter are possible.

5. The CFD-based optimization method of claim 4, wherein:
in the setting, the design variable comprises at least one of a rotational speed (rpm) of the impeller, a shape of the impeller, the number of impellers, a diameter of the impeller, and an installation height of the impeller.

6. The CFD-based optimization method of claim 4, wherein:
in the setting, a maximum height of the impeller is set within an operating volume height of the fermenter.

7. The CFD-based optimization method of claim 1, wherein:
the calculating of the secondary values comprises calculating a tertiary value, which is a ratio of the oxygen transfer coefficient per power ratio (kLa/power ratio), using the oxygen transfer coefficient and the power ratio.

8. The CFD-based optimization method of claim 7, wherein:
the finding of the optimal point comprises finding the optimum point based on a comparison value obtained by comparing the tertiary values before and after the variation.

9. A CFD-based optimization system for a fermenter for amino acid fermentation comprising:

a simulation unit configured to perform simulation by a CFD-based optimization method for a fermenter for amino acid fermentation;
a fermenter optimized by the simulation unit and applied to a field;
a monitoring sensor configured to monitor error data deviating from an optimal state of the fermenter during an operation; and

a control unit configured to compare whether the error data monitored by the monitoring sensor exceeds an allowable error range and to notify an administrator of a result of the comparison.

10. The CFD-based optimization system of claim 9, wherein:

the fermenter comprises
a first-stage impeller, a second-stage impeller, and a third-stage impeller installed on a shaft driven by a motor, spaced apart at predetermined intervals upward from a lower end,
a first baffle and a second baffle provided on both sides of a diametrically inner wall, and
a sparger provided below the first-stage impeller to blow air.

# FIG. 1

| Set objective variable, constraint variable, and design variable | ~ST1 |

↓

| Generate three-dimensional shape for flow region of impeller fermenter for each condition | ~ST2 |

↓

| Calculate primary value by computational fluid dynamics analysis for flow region based on three-dimensional shape information | ~ST3 |

↓

| Calculate secondary values, which oxygen transfer coefficient and power ratio, using primary value | ~ST4 |
| Calculate tertiary value using secondary values | ~ST41 |

↓

| Find optimal point by comparison value obtained by comparing secondary values before and after variation when varying design variable | ~ST5 |
| Find optimal point by comparison value obtained by comparing tertiary values before and after variation | ~ST51 |

# FIG. 2

FIG. 3

FIG. 4

## FIG. 5

## FIG. 6

# FIG. 7

## INTERNATIONAL SEARCH REPORT

| International application No. |
|---|
| **PCT/KR2023/011621** |

| A. | CLASSIFICATION OF SUBJECT MATTER |
|---|---|

**C12M 1/36**(2006.01)i; **C12M 1/06**(2006.01)i; **C12M 1/00**(2006.01)i

According to International Patent Classification (IPC) or to both national classification and IPC

| B. | FIELDS SEARCHED |
|---|---|

Minimum documentation searched (classification system followed by classification symbols)

C12M 1/36(2006.01); C02F 11/04(2006.01); C05F 17/02(2006.01); C12M 3/00(2006.01); C12M 3/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 아미노산(amino acid), 발효(fermentation), 전산유체역학(computational fluid dynamics), 시뮬레이션(simulation), 모니터링(monitoring)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y<br>A | JP 7094834 B2 (HITACHI ZOSEN INOVA AG) 04 July 2022 (2022-07-04)<br>See paragraphs [0032], [0046] and [0048]-[0050]; claim 1; and figure 1. | 9-10<br><br>1-8 |
| Y | ZOU, Xiang et al. Real-time fluid dynamics investigation and physiological response for erythromycin fermentation scale-up from 50 L to 132 m3 fermenter. Bioprocess Biosyst Eng. 2012, vol. 35, pp. 789-800.<br>See page 794; and table 1. | 9-10 |
| A | KR 10-2050846 B1 (LIM, Seung Taek) 02 December 2019 (2019-12-02)<br>See entire document. | 1-10 |
| A | KR 10-2004-0064260 A (BAYER HEALTHCARE AG) 16 July 2004 (2004-07-16)<br>See entire document. | 1-10 |
| A | MOILANEN, Pasi et al. Modeling Aerated Fermenters with Computational Fluid Dynamics. Ind. Eng. Chem. Res. 2006, vol. 45, pp. 8656-8663.<br>See entire document. | 1-10 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **13 December 2023** | **14 December 2023** |

| Name and mailing address of the ISA/KR | Authorized officer |
|---|---|
| **Korean Intellectual Property Office**<br>**Government Complex-Daejeon Building 4, 189 Cheongsa-**<br>**ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/011621**

| Patent document cited in search report | | | Publication date (day/month/year) | Patent family member(s) | | | Publication date (day/month/year) |
|---|---|---|---|---|---|---|---|
| JP | 7094834 | B2 | 04 July 2022 | CN | 109423442 | A | 05 March 2019 |
| | | | | EP | 3450536 | A1 | 06 March 2019 |
| | | | | EP | 3450536 | B1 | 08 April 2020 |
| | | | | JP | 2019-042733 | A | 22 March 2019 |
| | | | | US | 10519408 | B2 | 31 December 2019 |
| | | | | US | 2019-0062682 | A1 | 28 February 2019 |
| KR | 10-2050846 | B1 | 02 December 2019 | None | | | |
| KR | 10-2004-0064260 | A | 16 July 2004 | CN | 1578830 | A | 09 February 2005 |
| | | | | EP | 1451290 | A2 | 01 September 2004 |
| | | | | EP | 1451290 | B1 | 05 January 2011 |
| | | | | JP | 2005-501553 | A | 20 January 2005 |
| | | | | JP | 2009-089711 | A | 30 April 2009 |
| | | | | JP | 4279669 | B2 | 17 June 2009 |
| | | | | KR | 10-0909316 | B1 | 24 July 2009 |
| | | | | WO | 03-020919 | A2 | 13 March 2003 |
| | | | | WO | 03-020919 | A3 | 09 October 2003 |

Form PCT/ISA/210 (patent family annex) (July 2022)